# EUROPEAN PATENT SPECIFICATION

(11) **EP 1 591 518 B1**
(45) Date of publication and mention of the grant of the patent: **27.08.2008**
(21) Application number: 04703331.1
(22) Date of filing: 20.01.2004
(51) Int. Cl.: C07K 14/405, C12M 1/42

(54) **PROCESS FOR PREPARING PHYCOERYTHRIN WITH HIGH OPTICAL DENSITY**
VERFAHREN ZUR HERSTELLUNG VOM PHYCOERYTHRIN MIT HOHER OPTISCHEN DICHTE
PROCEDE DE PREPARATION DE PHYCOERYTHRINE A HAUTE DENSITE OPTIQUE

(30) Priority: 27.01.2003 CN 03102038; 27.01.2003 CN 03102039; 27.01.2003 CN 03102040; 27.01.2003 CN 03102041; 27.01.2003 CN 03236855; 27.01.2003 CN 03236856; 27.01.2003 CN 03236857; 27.01.2003 CN 03236858
(43) Date of publication of application: 02.11.2005
(73) Proprietor: Zen-u Biotechnology Co., Ltd, Taipei 231 (TW); Lu, Chihyi, Chiayi County 606 (TW)
(72) Inventor: Lu, Chihyi, Jhongpu Township, Chiayi 606,Taiwan (CN); Chiang, Youngmeng, Da-an District, Taipei 106, Taiwan (CN); Chou, Hongnong, Taipei, Taiwan (CN); Jeng, Jiunnming, Sinjhuang, Taipei County 242, Taiwan (CN)
(74) Representative: Winkler, Andreas Fritz Ernst
(86) International application number: PCT/CN2004/000075
(87) International publication number: WO 2004/067695

(56) References cited:
- FR-A- 2 690 452
- JP-A- 7 286 112
- US-A- 4 078 332
- US-A- 5 358 859

## Description

### BACKGROUND OF THE INVENTION

### 1. Field of the Invention

The present invention relates to a method for producing phycoerythrin with high optical density [OD], and more particularly to a method for producing phycoerythrin with high optical density [OD] using algae selected from the group consisting of Galaxaura oblongata, Halymenia ceylanica, Helminthocladia australis, and Porphyra dentata.

### 2. Description of the Prior Art

Natural pigment proteins from plants are safe when used in food and drink. The pigments are stable in mild heat, and acidic or basic solutions. Therefore they can be utilized in food and cosmetics as coloring agents. In addition to this, the pure form of the pigment can be used in fluorescent labeling of antibodies used as diagnostic agents in immunological, clinical, cell biological and biochemical research.

Phycocyanin and phycoerythrin are two currently used natural pigment proteins, and have been applied to many fields. As the major raw material for phycocyanin is blue-green algae such as Spirulina and Microcystis which is easy to grow and a large number of methods of algae cultivation and phycocyanin preparation have been developed thereby, the supply of phycocyanin does not cause a problem. However, there is still a scarcity of phycoerythrin and because of this, its price is high. The scarcity is due to shortages of raw material and difficulties in processing in connection with the commercial production of plycoelythrin.

Most phycoerythrin is extracted from red algae thalli such as Porphyra and Ceramium, and only a little from Porphyridium, which is now available from tank cultivation.

Although an increasing amount of wild red algae and cultivated Porphyra is utilized as raw material for phycoerythrin, most of it has high gel content, making the extraction of phycoerythrin from these plants very difficult, especially as regards dried algae. Furthermore, the quantity and quality of wild algae are influenced by the season and the ambient temperature. These elements make the production of phycoerythrin from wild and cultivated Porphyra even more difficult.

Extracting phycoerythrin from Porphyridium also has its difficulties, because the collection of single-cell growths is usually time-consuming and labor-intensive and the soluble polysaccharide secreted during the cultivation of algae deters cell collection and influences the extraction of phycoerythrin.

To solve the above problems, a process for preparing phycoerythrin from Bangia atropurpurea and Porphyra angusta is disclosed in US patent 5,358,858. Because these filamentous plants do not contain gel and can thus be maintained under controlled conditions such as culture media, temperature, degree and period of illumination. Phycoerythrin can be extracted from the filamentous plants easily. The process of the above invention includes the following steps:
1. Mature Bangia atropurpurea or Porphyra angusta thalli are collected from the sea and washed with sterilized seawater. After a short period of air-drying, they are placed in a culture medium (SWM-III medium, refer to Table 1). After a few hours, spores are released from Bangia atropurpurea or Porphyra angusta thalli. The released spores are then removed from the original medium and placed in a growth chamber where the temperature, degree of illumination, and daily period of said illumination are the ambient temperature, 1000 lux-4000 lux and 10-16 hours.

**Table 1**

| | | |
|---|---|---|
| NaNO3 | 8.5 g/100 ml | get 2ml |
| Na2HPO4 | 0.595 g/100 ml | get 2ml |
| Na2EDTA | 0.5 g/100 ml | get 2ml |
| | 0.01625 g/100 ml | get 2ml |
| FeCl3 (or FeCl3·7H2O) | 0.02885 g/100 ml | 2ml |
| *1PI-metal | | 2ml |
| *2S-3 Vitamins | | 2ml |
| Soil extract | | 50ml |
| Tris | (10 cc/l) | 500mg |
| Liver extract | | 10mg |
| Sea Water | up to pH=7.5 | 11 |
| (Obtain a thicker density of HCl of which the PH value is lower than 7 at first, then add NaOH solution to increase the PH value to about 7.5. This process will avoid the production of sediment) | | |
| *1PI-metal | | |
| H3BO3 | 12.368 g | |
| MnCl2 | 1.385 g | |
| ZnCl2 | 0.109 g add distilled water 21 | |
| CoCl2·6H2O | 4.479 mg | |
| CuCl2·2H2O | 0.034 mg | |
| *2S-3 Vitamins | | |
| Thiamin HC | 0.5 g | |
| Capantothenate | 0.1 g | |
| Nicotinic acid | 0.1 g | |
| P-aminobenzoic acid | 10 mg | |
| Biotin | 1 mg add distilled water 21 | |
| Inositol | 5 g | |
| Folic acid | 2 mg | |
| Thymine | 3 mg | |
| B12 | 1 mg | |

2. After the spores germinate into branched filaments, they are transferred to SWM-III flasks containing medium and cultivated in the above conditions until they form colonies. The filamentous colonies are then cut into small segments using a sterilized grinder and moved to a larger space, such as a tank, in order to facilitate further growth. After this they are transferred into a larger space and more filaments are generated. The filamentous colonies are cut up again for further growth until the required amount has grown. Note that when the filamentous colonies are cultivated in a large tank, fresh air (300 ml air/min) must be supplied to the tank. The filaments are then collected and filtered by a net of 100-400 mesh. The culture medium can be recovered and reused.
3. The collected and filtered Bangia atropurpurea or Porphyra angusta filaments are then fast dried in a vacuum or by warm air and ground into powder. The powder is added to a phosphate solution or water and mixed completely. Debris is removed by centrifugation to obtain a clear-red pigment solution. Crude phycoerythrin can then be obtained by adding (NH₄)₂SO₄ to make it a 20%-30% saturated solution so removing unnecessary proteins, followed by sedimentation with a 60%-65% (NH₄)₂SO₄ saturated solution. The phycoerythrin obtained has an OD₅₆₅/OD₂₈₀ of 1.4-1.6 and can be used for the production of food-grade and cosmetics-usable pigments.
4. The crude precipitated phycoerythrin can be further purified by gel filtration chromatography. For example, after purifying with Sephadex G200 chromatography once, the OD_{565/}OD₂₈₀ ratio of the produced phycoerythrin can reach 3.3-3.7. After repeating the purification process, the OD₅₆₅/OD280 ratio can reach 5.1-5.2. The purity of the phycoerythrin is about 99% when tested with SDS electrophoresis. This indicates that the phycoerythrin produced by the process of the invention can be used as reagents for immunoassaying.

Owing to phycoerythrin with the 5.1-5.2 value of OD₅₆₅/ OD₂₈₀ ratio being obtained through the complex purification processes twice in step 4 the manufacturing cost and time is increased. Therefore, we need to find a method that uses the filaments of other plants to produce phycoerythrin with high OD directly from the first clear-red pigment solution in step 3.

### SUMMARY OF THE INVENTION

In the light of the state of the art described above, it is an object of the present invention to provide a method for producing phycoerythrin with high optical density which is immune to the problems of the conventional process for preparing phycoerythrin from Bangia atropurpurea and Porphyra angusta described above.

It is another object of this invention to provide a method for producing phycoerythrin with higher optical density than the unit weight of the algae selected from the group consisting of Galaxaura oblongata, Halymenia ceylanica, Helminthocladia australis, and Porphyra dentata has plenty of weight of phycoerythrin to reduce the manufacturing cost.

It is a further object of this invention to provide a method for producing phycoerythrin with higher density than the first clear-red pigment solution of the algae selected from the group consisting of Galaxaura oblongata, Halymenia ceylanica, Porphyra dentata, and Helminthocladia australis has high OD phycoerythrin to reduce the number of manufacturing steps.

In view of the above and other considerations which will become apparent as the description proceeds, there is provided according to a general aspect of the present invention a method for producing phycoerythrin with high optical density (OD), which comprises the following steps: cultivating a gametophyte with mature tetrasporangia in a medium in order to obtain tetraspores; cultivating the tetraspores in conditions such that the temperature, light intensity and light/dark ratio are respectively 15-30°C, 500 lux-6000 lux and above 10:14 to germinate filaments; collecting the cultivated filaments; adding the cultivated filaments to a liquid solution with a pH value of 5-10; obtaining a clear-red pigment protein solution containing phycoerythrin by centrifuging the liquid solution at 6000 rpm for 10 minutes at 4°C; and salting out the gel-form phycoerythrin concentrate from the clear-red pigment protein solution, where the gametophyte is selected from an algae whose life cycle shows sexual reproduction, asexual reproduction, and vegetative propagation.

Based on the idea described above, wherein the algae is selected from the group consisting of Galaxaura oblongata, Halymenia ceylanica, Helminthocladia australis, and Porphyra dentata.

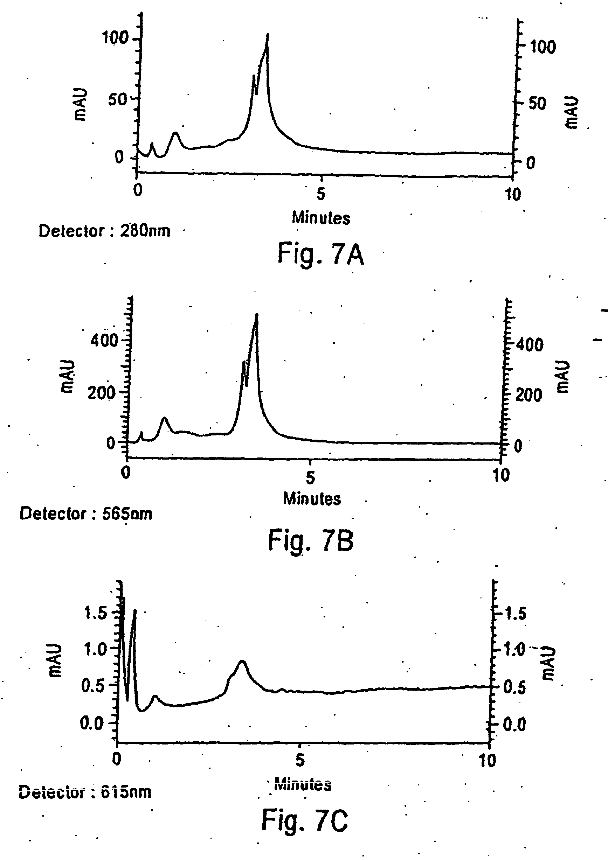

Based on the aforementioned idea, wherein chromatography spectrogram at 565 nm of phycoerythrin extracted from the cultivated filaments of Galaxaura oblongata carpospores measured by High Performance Liquid Chromatography (HPLC) is shown as in Figure 7B.

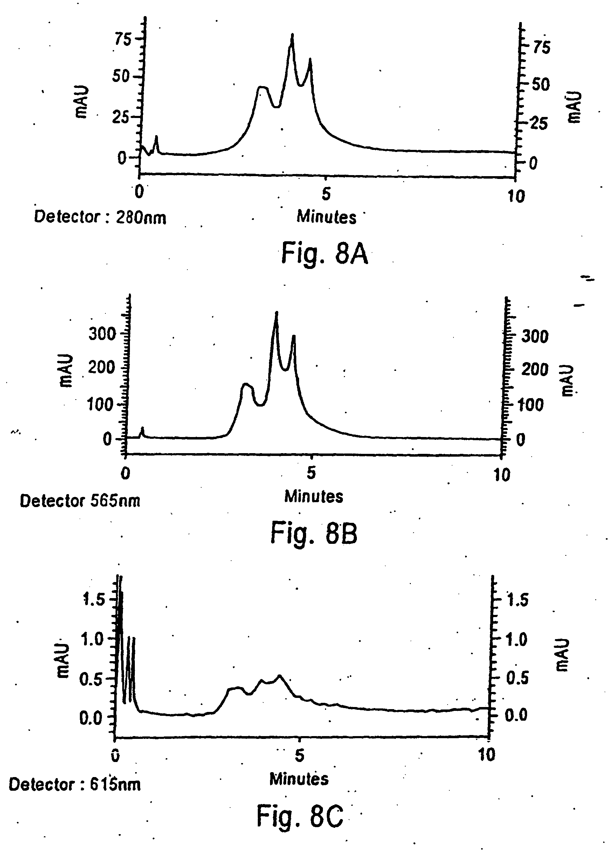

Based on the idea described above, wherein the chromatography spectrogram at 565 nm of phycoerythrin extracted from the cultivated filaments of Halymenia ceylanica carpospores measured by High Performance Liquid Chromatography (HPLC) is shown as in Figure 8B.

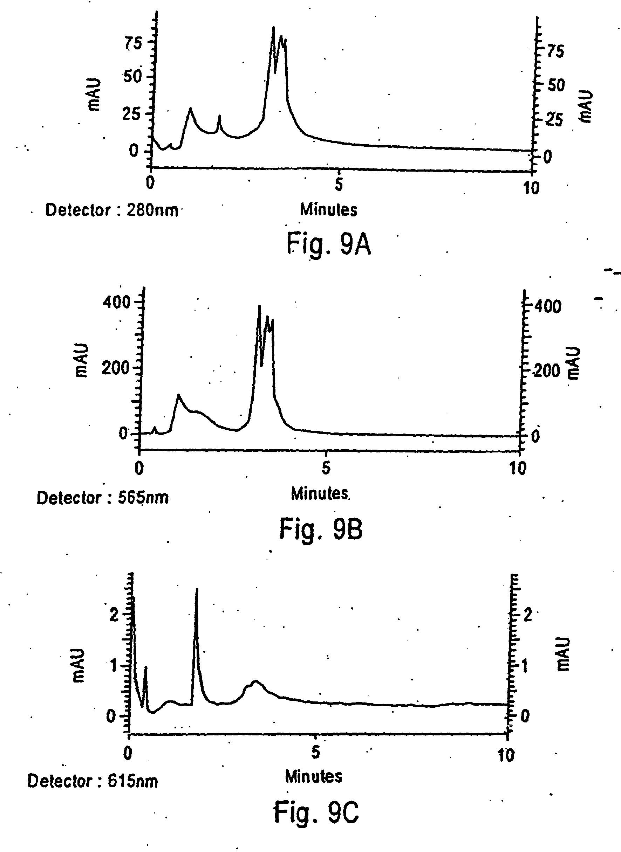

Based on the aforementioned idea, wherein the chromatography spectrogram at 565 nm of phycoerythrin extracted from the cultivated filaments of Helminthocladia australis carpospores measured by High Performance Liquid Chromatography (HPLC) is shown as in Figure 9B.

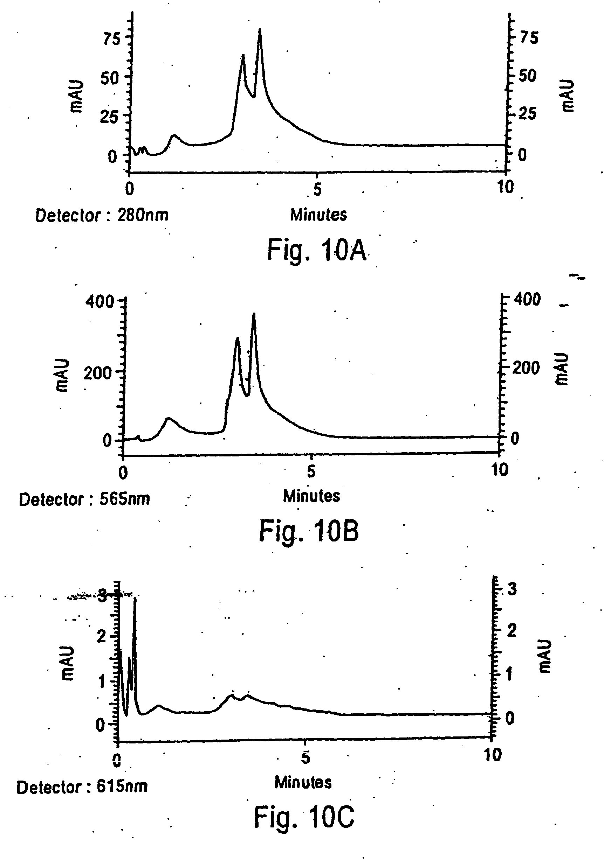

Based on the idea described above, wherein the chromatography spectrogram at 565 nm of phycoerythrin extracted from the cultivated filaments of Porphyra dentata carpospores measured by High Performance Liquid Chromatography (HPLC) is shown as in Figure 10B.

Based on the aforementioned idea, wherein the medium is a SWM-III medium.

Based on the idea described above, wherein the SWM-III medium is an inorganic SWM-III medium.

Based on the aforementioned idea, wherein the step of cultivating the tetraspores to germinate filaments further involves breaking up the filaments into minute segments and cultivating them in a larger tank in the same conditions until the cultivated filaments grow to the required amounts, wherein the tank is supplied with fresh air for keeping the minute segments suspended in the medium.

Based on the idea described above, wherein the improved conditions such as temperature, light intensity and light/dark ratio are respectively 20°C, 2000 lux, and 12:12.

Base on the aforementioned idea, wherein the step of collecting the cultivated filaments further involves collecting the cultivated filaments by a net of 20-400 mesh, drying the cultivated filaments, and grinding the cultivated filaments into powder.

Based on the idea described above, wherein the method of drying the cultivated filaments is selected from the group consisting of the vacuum method or the warm air method.

Based on the aforementioned idea, wherein the liquid solution consists of water and potassium phosphate.

Based on the idea described above, wherein the step of salting out the gel-form phycoerythrin further involves adding the 20% solution of (NH₄)₂SO₄ to the clear-red pigment protein solution, and centrifuging the clear-red pigment protein solution at 6000 rpm for 10 minutes at 4°C to separate the unwanted proteins and so obtain a purer pigment protein solution.

Based on the idea described above, wherein the step of salting out the gel-form phycoerythrin further involves adding the 60-65% solution of (NH₄)₂SO₄ to the purer pigment protein solution, and centrifuging the purer pigment protein solution at 6000 rpm for 10 minutes at 4°C to obtain the gel-form phycoerythrin concentrate.

Based on the aforementioned idea, wherein the step of salting out the gel-form phycoerythrin further involves dialyzing the gel-form phycoerythrin concentrate and purifying the phycoerythrin by gel filtration thereof.

Based on the idea described above, wherein the gel filtration is a Sephadex G200 gel filtration.

Based on the aforementioned idea, wherein the step of salting out the gel-form phycoerythrin further involves purifying the gel-form phycoerythrin concentrate using ultra-filtration.

### BRIEF DESCRIPTION OF THE DRAWINGS

The foregoing aspects and many of the attendant advantages of this invention will become more readily appreciated as the same becomes better understood by reference to the following detailed description, when taken in conjunction with the accompanying drawings, wherein:
Fig. 1 shows the chromatography spectrogram of absorption and emission of phycoerythrin measured by High Performance Liquid Chromatography (HPLC);
Fig. 2 shows the life cycle of Bangia atropurpurea;
Fig. 3 shows the life cycle of Porphyra angusta;
Fig. 4 shows the life cycle of Nemalion;
Figs. 5A∼5C shows the chromatography spectrogram of phycoerythrin extracted from Bangia atropurpurea measured by HPLC at 280 nm, 565 nm, and 615 nm;

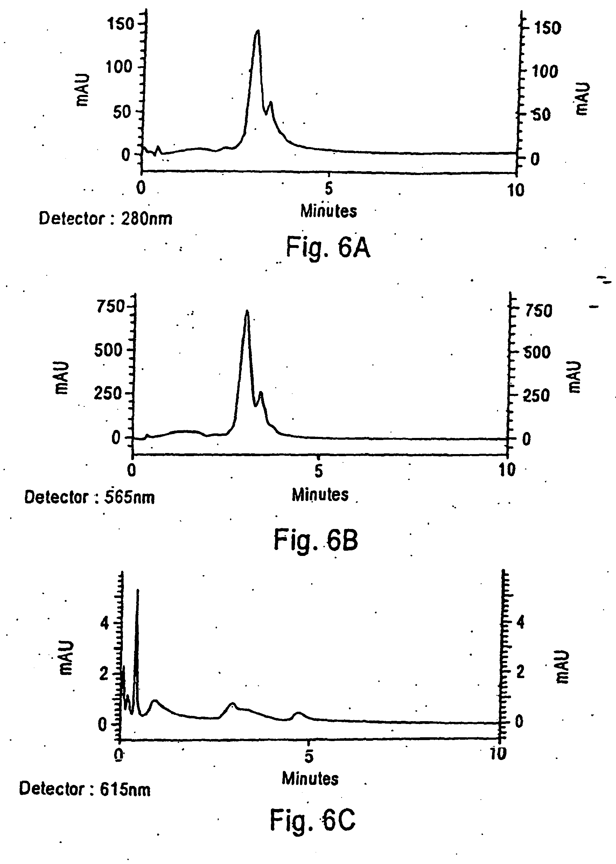

Figs. 6A∼6C shows the chromatography spectrogram of phycoerythrin extracted from Porphyra angusta measured by HPLC at 280 nm, 565 nm, and 615 nm;
Figs. 7A∼7C shows the chromatography spectrogram of phycoerythrin extracted from Galaxaura oblongata measured by HPLC at 280 nm, 565 nm, and 615 nm;
Figs. 8A∼8C shows the chromatography spectrogram of phycoerythrin extracted from Halymenia ceylanica measured by HPLC at 280 nm, 565 nm, and 615 nm;
Figs. 9A∼9C shows the chromatography spectrogram of phycoerythrin extracted from Helminthocladia australis measured by HPLC at 280 nm, 565 nm, and 615 nm; and
Figs. 10A∼10C shows the chromatography spectrogram of phycoerythrin extracted from Porphyra dentata measured by HPLC at 280 nm, 565 nm, and 615 nm.
Figs. 11∼15 show the elements of the device of the present invention for producing phycoerythrin with high optical density [OD].

### DESCRIPTION OF THE PREFERRED EMBODIMENT

Some sample embodiments of the present invention will now be described in greater detail. Nevertheless, it should be recognized that the present invention can be practiced in a wide range of other embodiments besides those explicitly described, and the scope of the present invention is expressly not limited except as specified in the accompanying claims.

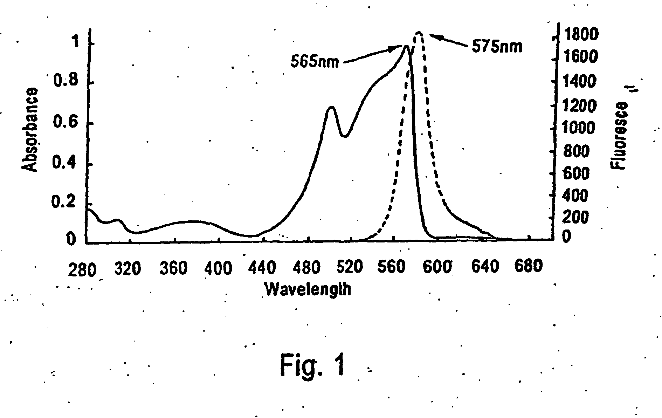

Phycobiliproteins are water-soluble fluorescent pigment proteins derived from algae. They are widely used in the fluorescent labeling of antibodies that are applied as diagnostic agents owing to their special fluorescent properties. Phycoerythrin has the highest fluorescent intensity among Phycobiliproteins, so it is used in many fluorescent tests. The chromatography spectrogram of absorption and emission of phycoerythrin measured by High Performance Liquid Chromatography (HPLC) is shown as Fig. 1. The chromatography conditions are as below:
HPLC column: HYDROCELL DEAE NP10
Column size: 50*4.6mm
Buffer A: 10mMK-PBS pH6.0
Buffer B:10mMk-PBS ,0.5M NaCl pH6.0
Gradient:0% Buffer B→ 12min→ 50% Buffer B
Detection:565nm
Flow rate: 1ml/min
HPLC is brought about by use of pump accessories, filters, detectors, and a recorder.

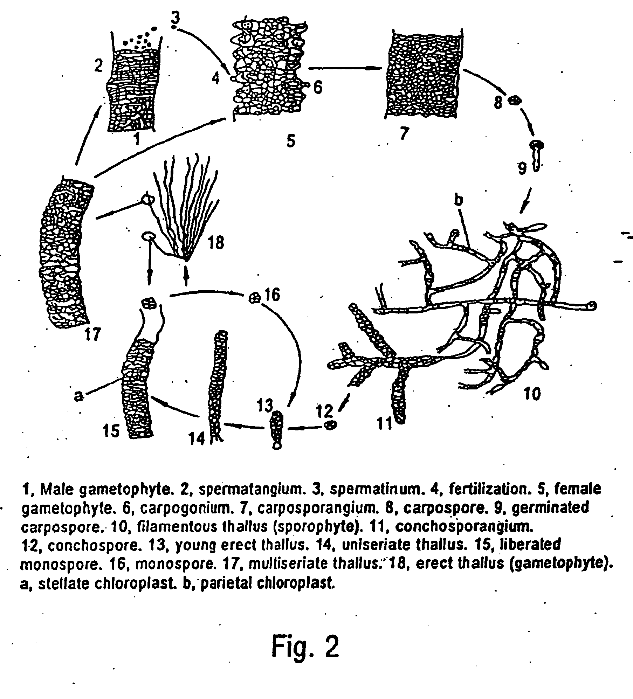

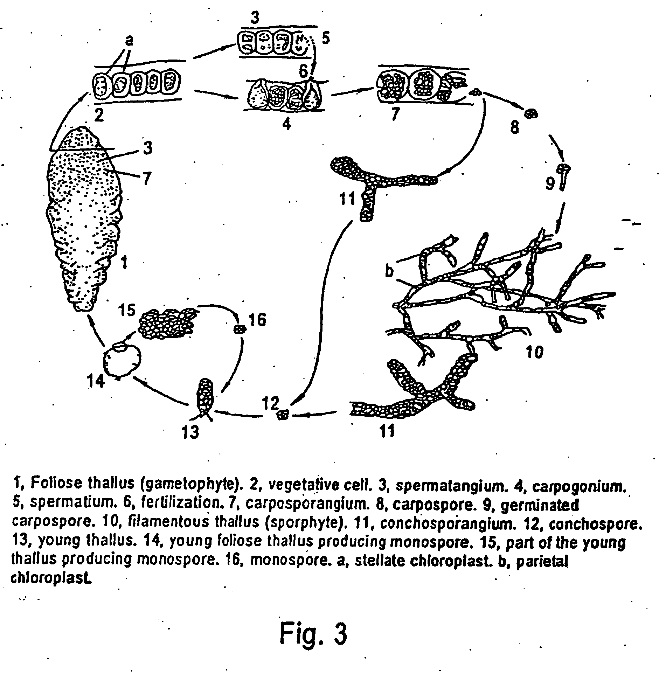

Proceeding generations alternate between sexual reproduction and asexual reproduction in the life cycles of Bangia atropurpurea and Porphyra angusta, as shown in Figs. 2 and 3. A mature male gametophyte produces spermatiums in the spermatangiums. These spermatiums are released into the water and are carried by currents to the carpogoniums of female gametophytes. A mature carpogonium produces carpospores to form filamentous thallus (sporophyte) with conchosporangia, and the conchospores are released from the mature conchosporangium to form young erect thalli. After a time, the young erect thalli release the monospores to form new young erect thalli repeatedly or the erect thalli (gametophytes) in the proper conditions. In this way, the alternating of sexual reproduction and asexual reproduction in proceeding generations continues.

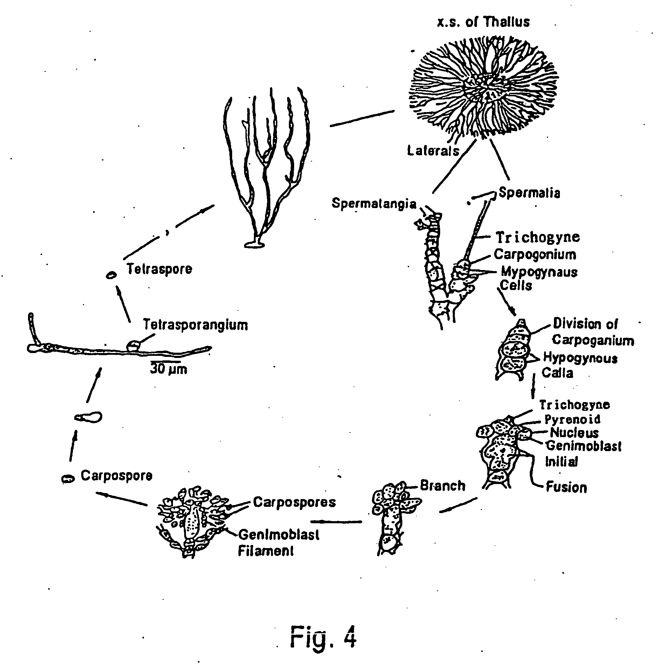

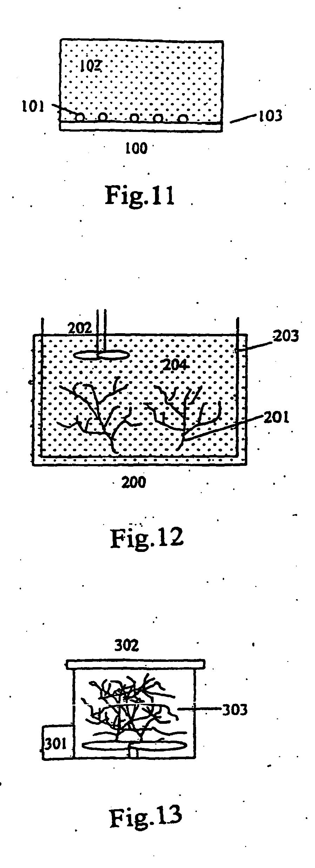

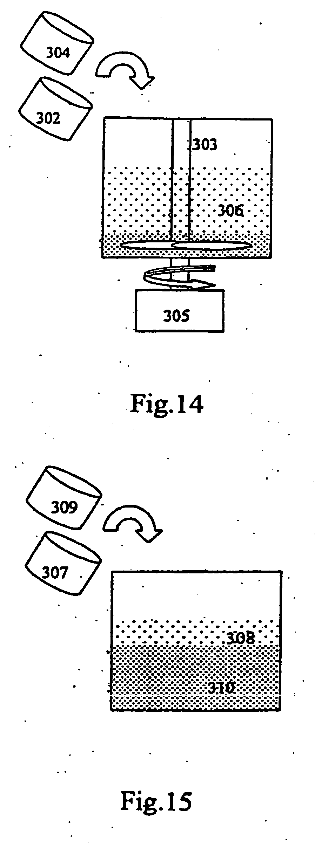

In other algae, such as Nemalion shown in Fig. 4, generations alternate between sexual reproduction, asexual reproduction and vegetative propagation in their life cycles of. A mature carpogonium produces carpospores to form tetrasporophytes with tetrasporangia, and the tetraspores are released from the mature tetrasporangium to form filamentous thallus. The filamentous thallus of the tetraspore and the filamentous thallus of the carpospore have different properties. Similarly, the filamentous thallus of the tetraspore does not contain gel and thus can be maintained under certain controlled conditions such as second medium, temperature, degree of illumination, and a daily period of said illumination. A method of extracting Phycoerythrin with high OD easily from the filamentous thallus of tetraspore is disclosed in this invention. The specific algae can be selected from Galaxaura oblongata, Halymenia ceylanica, Helminthocladia australis, Porphyra dentate or any combination thereof. For exact details, please refer to Figs. 11 to.15, where the Galaxaura oblongata will be the example introduced. The process and the device of the prevent invention is disclosed as follows:
1. The gametophytes with mature tetrasporangia selected from algae whose life cycle includes sexual reproduction, asexual reproduction, and vegetative propagation, such as Galaxaura oblongata, Halymenia ceylanica, Helminthocladia australis, and Porphyra dentata, are collected and washed with sterilized water by using a first medium (not shown). After a short period of air-drying, they are placed into a second medium 100 (inorganic SWM-III medium). After a few hours, tetraspores 101 are released from the algae. The released tetraspores 101 are then removed from the second medium and placed in a growth chamber, such as a sheet glass 103, wherein the temperature, degree of illumination, light/dark ratio and daily period of said illumination are respectively 15-30°C, 500 lux-6000 lux, above 10:14, and 10-16 hours every day. But a more effective temperature, degree of illumination and light/dark ratio for the procedure are respectively 20°C, 2000 lux, and 12:12. Please refer to Fig.11.
2. After the tetraspores germinate into branched filaments 201, the filaments 201 (not shown) are collected and transferred to an inorganic SWM-III medium-containing flask, such as a tank 200, and cultivated in the above conditions until they form colonies. The filamentous colonies are then cut into small segments using a sterilized grinder and moved to a larger space in order to facilitate further growth. After they are transferred into a larger space, more filaments are generated. The filamentous colonies are cut again for further growth until the required amount is acquired. Note that when the filamentous colonies are cultivated in a large tank, fresh air (300 ml air/min) must be supplied to the tank by a pump 202 to allow the colonies to be suspended in the medium 204. The filaments are then collected and filtered by a net 203 of 20-400 mesh. The culture medium can be recovered and reused. Please refer to **Fig.12****.**
3. Then the filtered filaments 300 are collected using the floating cultivating process and are then fast dried by a drier , such as a vacuum or warm air device 301, and ground into powder by a grinder 302. Please refer to Fig.13. The powder, for example 2.4 gram powder, is added to a solution with a pH value of 5-10, such as 70 ml and 10mM potassium phosphate 304 or any kind of liquids, and mixed completely by a mixer 303. After centrifugation by a centrifugation device 305 at 6000 rpm for 10 minutes at 4°C, a clear-red pigment solution will be obtained. Please refer to Fig.14. Crude phycoerythrin can then be obtained by adding 20%-30% (NH₄)₂SO₄ saturated solution 307 to remove unnecessary protein sediment 308 by a settler (not shown), and adding 60%-65% (NH₄)₂SO₄ saturated solution 309 by sedimentation to obtain the crude phycoerythrin with high OD which may be used to produce food-grade and cosmetics-usable pigments 310. The OD₅₆₅/OD₂₈₀ of the pigment 310 is 2.66 by testing. Please refer to Fig.15. The above mentioned clear-red pigment solution can also be obtained by grinding wet filaments directly and adding potassium phosphate thereto. The pigment can also be produced by an ultra-filtration process.
4. The crude precipitated phycoerythrin can be further purified by gel filtration chromatography or ultra-filtration. For example, after purifying with Sephadex G200 chromatography once, the OD₅₆₅ /OD₂₈₀ ratio of the produced phycoerythrin reaches 4.5. After a repeated purification process, the OD₅₆₅ /OD₂₈₀ ratio of phycoerythrin reaches 5.3. The purity of the phycoerythrin is about 99% when tested with SDS electrophoresis. This indicates that the phycoerythrin produced by the process of the invention can be used as a reagent for immunoassay.

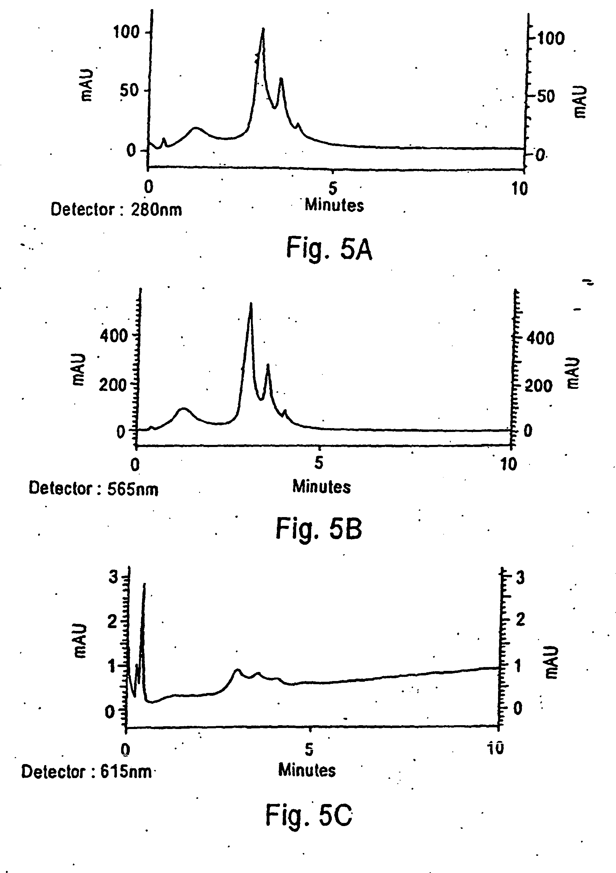

The chromatography spectrogram of phycoerythrin extracted from Bangia atropurpurea and Porphyra angusta measured by HPLC at 280 nm, 565 nm, and 615 nm are shown in Figs. 5A∼6C. Figs. 7A∼10C show the chromatography spectrogram of phycoerythrin extracted from Galaxaura oblongata, Halymenia ceylanica, Helminthocladia australis, and Porphyra dentata measured by HPLC at 280 nm, 565 nm, and 615 nm respectively. The phycoerythrin extracted from each alga has a special chromatography spectrogram, so we can know easily the source of phycoerythrin by HPLC.

There are the OD values in Table 2 for the six algae that comprise Bangia atropurpurea, Porphyra angusta, Galaxaura oblongata, Halymenia ceylanica, Helminthocladia australis, and Porphyra dentata. The first row of the table 2 is shows the quantity (milligram) of phycoerythrin produced based on the same quantity (gram) of algae. The second row and the third row show the OD₅₆₅/OD₂₈₀ ratio and the OD₆₁₅/OD₅₆₅ ratio compared value between the algae used in the present invention and Bangia atropurpurea and Porphyra angusta. The higher the former value, the higher the purity of phycoerythrin obtained. The high purity of phycoerythrin thus obtained is sufficient to produce food-grade and cosmetics-usable pigments. It also can be used in immunity treatments for the prevention of disease. The latter value is shown as a ratio of purity of phycocyanin and phycoerythrin. The lower the latter value, the lower the purity of phycocyanin obtained, that means, the higher the quality of the phycoerythrin produced. Hence, the filaments of the algae used in the present invention, such as Galaxaura oblongata, Halymenia ceylanica, Helminthocladia australis, and Porphyra dentata can be utilized to obtain higher OD value phycoerythrin than the prior art.

**Table 2**

| algae | Ba | Pa | Go | Hc | Ha | Pd |
|---|---|---|---|---|---|---|
| RPE (mg) / algae (g) | 53.5 | 38.89 | 57.74 | 46.59 | 48.1 | 44.98 |
| OD₅₆₅/OD₂₈₀ | 1.40 | 1.54 | 2.66 | 1.44 | 2.34 | 1.96 |
| OD_{615/}OD₅₆₅ | 0.19 | 0.53 | 0.14 | 0.10 | 0.15 | 0.21 |

| | | | | | | |
|---|---|---|---|---|---|---|
| R P E : a phycoerythrin protein with maximum UV absorbtion wavelength 566nm and fluorescence emission wavelength 575nm. Ba : Bangia atropurpurea Pa : Porphyra angusta Go : Galaxaura oblongata Hc : Halymenia ceylanica Ha : Helminthocladia australis Pd : Porphyra dentata | | | | | | |

Although the specific embodiment has been illustrated and described, it will be obvious to those skilled in the art that various modifications may be made without departing from what is intended to be limited solely by the appended claims.

## Claims

1. A method for producing phycoerythrin with high optical density (OD), utilizing a tetraspore filament of a specific algae whose life cycle has sexual reproduction, asexual reproduction, and vegetative propagation to produce phycoerythrin with high optical density (OD), wherein the specific algae is selected from the group consisting of Galaxaura oblongata, Halymenia ceylanica, Helminthocladia australis, Porphyra dentate and the combination thereof.

2. The method according to claim 1, wherein the tetraspore filament is a carpospore filament.

3. The method according to claim 1 or 2, the steps of the method for producing phycoerythrin with high optical density (OD) comprising:
cultivating a gametophyte with mature tetrasporangia in a medium to obtain tetraspores therefrom;
cultivating the tetraspores in conditions such that the temperature, degree of illumination and light/dark ratio are respectively 15-30°C, 500 lux - 6000 lux and above 10:14 to germinate filaments;
collecting the cultivated filaments;
adding the cultivated filaments to a liquid solution with a pH value of 5-10;
obtaining a clear-red pigment protein solution containing phycoerythrin by centrifuging the liquid solution; and
salting out the concentrated gel-form phycoerythrin from the clear-red pigment protein solution.

4. The method according to claim 1, wherein the medium is a SWM-III medium or an inorganic SWM-III medium.

5. The method according to claim 3, wherein collecting the cultivated filaments is by using a floating cultivating process of which the temperature, degree of illumination and light/dark ratio are respectively 20°C, 2000 lux, and 12:12.

6. The method according to claim 1, wherein the step of collecting the cultivated filaments further comprises:
collecting the cultivated filaments by a net;
drying the cultivated filaments; and
grinding the cultivated filaments into powder.

7. The method according to claim 6, wherein the net is selected from 20 to 400 mesh.

8. The method according to claim 6, wherein the method of drying the cultivated filaments is selected from the group consisting of the vacuum method or the warm air method.

9. The method according to claim 3, wherein the condition of centrifuging is at 6000 rpm for 10 minutes, at 4°C.

10. The method according to claim 3, wherein the liquid solution consists of water and potassium phosphate.

11. The method according to claim 3, wherein the step of salting out the gel-form phycoerythrin further comprises:
adding the 20% solution of (NH₄)₂SO₄ to the clear-red pigment protein solution; and
centrifuging the clear-red pigment protein solution at 6000 rpm for 10 minutes at 4°C to separate the unwanted proteins in order to obtain a purer pigment protein solution.

12. The method according to claim 3, wherein the step of salting out the gel-form phycoerythrin further comprises:
adding the 60∼65% solution of (NH₄)₂SO₄ to the purer pigment protein solution; and
centrifuging the purer pigment protein solution at 6000 rpm for 10 minutes at 4°C to obtain the gel-form phycoerythrin concentrate.

13. The method according to claim 3, wherein the step of salting out the gel-form phycoerythrin further comprises:
purifying the gel-form phycoerythrin concentrate by ultra-filtration thereof.

14. The method according to claim 3, wherein the step of salting out the gel-form phycoerythrin further comprises:
dialyzing the gel-form phycoerythrin concentrate and purifying the phycoerythrin by gel filtration thereof.

## Patentansprüche

1. Verfahren zur Herstellung von Phycoerythrin mit hoher optischer Dichte (OD) unter Verwendung eines Tetrasporen-Filamentes einer spezifischen Alge, deren Lebenszyklus sexuelle Reproduktion, asexuelle Reproduktion und vegetative Vermehrung aufweist, um Phycoerythrin mit hoher optischer Dichte (OD) herzustellen, wobei die spezifische Alge ausgewählt ist aus der Gruppe, bestehend aus Galaxaura oblongata, Halymenia ceylanica, Helminthocladia australis, Porphyra dentate und der Kombination davon.

2. Verfahren nach Anspruch 1, wobei das Tetrasporen-Filament ein Carposporen-Filament ist.

3. Verfahren nach Anspruch 1 und 2, wobei die Schritte des Verfahrens zur Herstellung von Phycoerythrin mit hoher optischer Dichte (OD):
Kultivieren eines Gametophyten mit reifen Tetrasporangia in einem Medium, um Tetrasporen daraus zu erhalten;
Kultivieren der Tetrasporen bei solchen Bedingungen, dass die Temperatur, der Belichtungsgrad und das Licht/Dunkel-Verhältnis 15-30°C, 500 Lux - 6000 Lux bzw. über 10:14 sind, um Filamente zum Keimen zu bringen;
Sammeln der kultivierten Filamente;
Zugeben der kultivierten Filamente zu einer flüssigen Lösung mit einem pH-Wert von 5-10;
Erhalten einer klar-roten Pigment-Proteinlösung, die Phycoerythrin enthält, durch Zentrifugieren der flüssigen Lösung; und
Aussalzen des konzentrierten Gelform-Phycoerythrins aus der klar-roten Pigment-Proteinlösung
umfassen.

4. Verfahren nach Anspruch 1, wobei das Medium ein SWM-III-Medium oder ein anorganisches SWM-III-Medium ist.

5. Verfahren nach Anspruch 3, wobei das Sammeln der kultivierten Filamente durch Verwendung eines Flotationskultivierungsverfahrens erfolgt, dessen Temperatur, Belichtungsgrad und Licht/Dunkel-Verhältnis 20°C, 2000 Lux bzw. 12:12 sind.

6. Verfahren nach Anspruch 1, wobei der Schritt des Sammelns der kultivierten Filamente weiter:
Sammeln der kultivierten Filamente mit einem Netz;
Trocknen der kultivierten Filamente; und
Zerkleinern der kultivierten Filamente zu Pulver
umfasst.

7. Verfahren nach Anspruch 6, wobei das Netz ausgewählt ist aus 20 bis 400 mesh.

8. Verfahren nach Anspruch 6, wobei das Verfahren des Trocknens der kultivierten Filamente ausgewählt ist aus der Gruppe, bestehend aus dem Vakuumverfahren oder dem Warmluftverfahren.

9. Verfahren nach Anspruch 3, wobei die Bedingung des Zentrifugierens bei 6000 UPM für 10 Minuten bei 4°C ist.

10. Verfahren nach Anspruch 3, wobei die flüssige Lösung aus Wasser und Kaliumphosphat besteht.

11. Verfahren nach Anspruch 3, wobei der Schritt des Aussalzens des Gelform-Phycoerythrins weiter:
Zugeben der 20%-igen Lösung von (NH₄)₂SO₄ zur klar-roten Pigment-Proteinlösung; und
Zentrifugieren der klar-roten Pigment-Proteinlösung bei 6000 UPM für 10 Minuten bei 4°C, um die unerwünschten Proteine abzutrennen, um eine reinere Pigment-Proteinlösung zu erhalten,
umfasst.

12. Verfahren nach Anspruch 3, wobei der Schritt des Aussalzens des Gelform-Phycoerythrins weiter:
Zugeben der 60-65%-igen Lösung von (NH₄)₂SO₄ zur reineren Pigment-Proteinlösung; und
Zentrifugieren der reineren Pigment-Proteinlösung bei 6000 UPM für 10 Minuten bei 4°C, um das Gelform-Phycoerythrin-Konzentrat zu erhalten,
umfasst.

13. Verfahren nach Anspruch 3, wobei der Schritt des Aussalzens des Gelform-Phycoerythrins weiter:
Reinigen des Gelform-Phycoerythrin-Konzentrats durch Ultrafiltration desselben
umfasst.

14. Verfahren nach Anspruch 3, wobei der Schritt des Aussalzens des Gelform-Phycoerythrins weiter:
Dialysieren des Gelform-Phycoerythrin-Konzentrats und Reinigen des Phycoerythrins durch Gelfiltration desselben
umfasst.

## Revendications

1. Procédé de préparation de phycoérythrine à haute densité optique (DO), en utilisant un filament tétraspore d'une algue spécifique dont le cycle de vie a une reproduction sexuée, une reproduction asexuée et une multiplication végétative pour produire la phytoérythrine à haute densité optique (DO), dans lequel l'algue spécifique est choisie dans le groupe comprenant Galaxaura oblongata, Halymenia ceylanica, Helminthocladia australis, Porphyra dentate et les combinaisons de celles-ci.

2. Procédé selon la revendication 1, dans lequel le filament tétraspore est un filament carpospore.

3. Procédé selon la revendication 1 ou 2, les étapes du procédé de production de phycoérythrine à haute densité optique (DO) étant les suivantes :
cultiver un gamétophyte avec un tétrasporange mature dans un milieu pour obtenir des tétraspores ;
cultiver les tétraspores dans des conditions telles que la température, le degré d'éclairage et le rapport lumière/obscurité soient respectivement de 15-30 °C, 300 lux - 6000 lux et supérieur à 10:14 pour faire germer les filaments ;
collecter les filaments cultivés ;
ajouter les filaments cultivés à une solution liquide ayant une valeur de pH de 5-10 ;
obtenir une solution de protéine pigment rouge clair contenant de la phycoérythrine en centrifugeant la solution liquide ; et
relarguer la phycoérythrine sous forme de gel concentré à partir de la solution de protéine pigment rouge clair.

4. Procédé selon la revendication 1, dans lequel le milieu est un milieu SWM-III ou un milieu SWM-III inorganique.

5. Procédé selon la revendication 3, dans lequel la collecte des filaments cultivés est opérée en utilisant un processus de culture flottante dont la température, le degré d'éclairage et le rapport lumière/obscurité sont respectivement de 20 °C, 2000 lux et 12:12.

6. Procédé selon la revendication 1, dans lequel l'étape de collecte des filaments cultivés comprend en outre les étapes suivantes :
collecter les filaments cultivés au moyen d'un filet ;
sécher les filaments cultivés ; et
broyer les filaments cultivés en poudre.

7. Procédé selon la revendication 6, dans lequel le filet est choisi à une maille de 20 à 400.

8. Procédé selon la revendication 6, dans lequel le procédé de séchage des filaments cultivés est choisi dans le groupe comprenant le procédé par le vide ou le procédé à l'air chaud.

9. Procédé selon la revendication 3, dans lequel la condition de centrifugation est à 6000 trs/min pendant 10 minutes, à 4 °C.

10. Procédé selon la revendication 3, dans lequel la solution liquide comprend de l'eau et du phosphate de potassium.

11. Procédé selon la revendication 3, dans lequel l'étape de relargage de la phycoérythrine sous forme de gel comprend en outre les étapes suivantes :
ajouter la solution à 20 % de (NH₄)₂SO₄ à la solution de protéine pigment rouge clair ; et
centrifuger la solution de protéine pigment rouge clair à 6000 trs/min pendant 10 minutes à 4 °C pour séparer les protéines indésirables afin d'obtenir une solution de protéine pigment plus pure.

12. Procédé selon la revendication 3, dans lequel l'étape de relargage de la phycoérythrine sous forme de gel comprend en outre les étapes suivantes :
ajouter la solution à 60-65 % de (NH₄)₂SO₄ à la solution de protéine pigment plus pure ; et
centrifuger la solution de protéine pigment plus pure à 6000 trs/min pendant 10 minutes à 4 °C pour obtenir le concentrat de phycoérythrine sous forme de gel.

13. Procédé selon la revendication 3, dans lequel l'étape de relargage de la phytoérythrine sous forme de gel comprend en outre l'étape suivants :
purifier le concentrat de phytoérythrine sous forme de gel par ultrafiltration de celui-ci.

14. Procédé selon la revendication 3, dans lequel l'étape de relargage de la phycoérythrine sous forme de gel comprend en outre l'étape suivants :
dialyser le concentrat de phycoérythrine sous forme de gel et purifier la phycoérythrine par filtration sur gel de celui-ci.
